# EUROPEAN PATENT SPECIFICATION

(11) **EP 4 452 354 B1**
(45) Date of publication and mention of the grant of the patent: **03.09.2025**
(21) Application number: 22834911.4
(22) Date of filing: 08.12.2022
(51) Int. Cl.: A61M 1/06, A61M 1/00

(54) **A BREAST PUMP**
MILCHPUMPE
TIRE-LAIT

(30) Priority: 20.12.2021 EP 21215871; 13.09.2022 EP 22195355
(43) Date of publication of application: 30.10.2024
(73) Proprietor: Koninklijke Philips N.V., 5656 AG Eindhoven (NL)
(72) Inventor: VAN DER KOOI, Johannes Tseard, 5656 AG Eindhoven (NL); LIPSCH, Job, 5656 AG Eindhoven (NL); BROCKHUIS, Lili-Marjan, 5656 AG Eindhoven (NL)
(74) Representative: Philips Intellectual Property & Standards
(86) International application number: PCT/EP2022/084896
(87) International publication number: WO 2023/117445

(56) References cited:
- WO-A1-2008/127991
- US-A1- 2017 065 753
- US-A1- 2021 121 614

## Description

### FIELD OF THE INVENTION

This invention relates to breast pumps.

### BACKGROUND OF THE INVENTION

Breast pumps are used by breast feeding women to extract milk from their breast such that the extracted milk can be fed to their babies at a later time.

It is well known that the best nutrition for babies is breast milk. The world health organization (WHO) recommends to breast feed babies for at least one year, preferably longer. However, mothers often go back to work after only several weeks or months. To provide the best nutrition to their babies, mothers may then express milk using a breast pump. The expressed milk can be stored and given to the baby at a later stage and/or by somebody else.

To use a breast pump, the breast is typically placed into a funnel-shaped cup and a vacuum is applied such that milk is extracted. A motorized breast pump typically has two operating modes, namely a stimulation mode and an extraction mode. The operation of a typical breast pump makes use of a cyclic pressure waveform, typically a negative pressure or vacuum, which is applied to the breast and nipple within a breast shield (or a pair of breast shields) to draw the milk from the nipple into a collection container. The typical pressure profiles oscillate between a maximum negative pressure and then return to atmospheric pressure at the end of each cycle.

During the stimulation mode, the milk ejection reflex is stimulated. This for example makes use of a relatively high frequency cyclic pressure waveform such as 2Hz. Once milk comes out of the breast it is advised to switch to the extraction mode, which has a lower frequency (typically around 1Hz) and higher intensity pumping (i.e. lower under pressure) for more effective milk extraction.

When the breast pump is activated, a pressure source such as a vacuum pump inside the breast pump generates a vacuum and the stimulation mode can be started. The vacuum pump is typically driven by an electric motor. For portable breast pumps, these motors are driven by battery power. There are also breast pumps using manually driven mechanical pumps.

A return to ambient pressure within the breast shield as part of the cyclic pressure waveform may not be desired, as it is known from research that babies maintain a baseline vacuum. The function of this baseline vacuum in a baby mouth is to keep a good latch on (i.e. keep the nipple at a stable position). Thus, benefits may be achieved by also maintaining a minimum level of vacuum on the breast throughout at least a portion of the pumping session when using a breast pump. In addition, there is discomfort when extracting the milk because of the relatively large back and forward movement of the nipple, caused by the alternating vacuum that is applied, which results in a continuous stretching and un-stretching of the nipple tissue. The use of a baseline pressure can also reduce this discomfort by keeping the nipple in a more stretched position.

Recently, there is a trend towards wearable breast pumps. These devices generally have a smaller form factor and can be fully worn on the user's body, allowing the user to move around freely. Some of these are small enough to fit into a user's bra. A motor, battery and milk collection container are for example integrated to form a single unit.

It is desirable particularly (but not exclusively) for wearable breast pumps that the device can be used in a hands free way, and a baseline pressure can assist this by providing continuous holding force.

WO 2008/127991 A1 discloses a breast pump that includes a mechanism to regulate the pressure change within a breast shield chamber, including in some cases to a maintained minimum pressure that is less than ambient (atmosphere) i.e. a baseline vacuum. The pressure regulator provides control for varying negative pressure or to achieve a specific measured negative pressure value within the breast shield. However, a complex mechanism is needed to maintain the baseline vacuum, and in addition the pressure difference between the breast shield and the container may prevent that milk can flow to the milk collection container. As a result, a complex valve and pressure control arrangement is needed.

It is also desirable to maintain separation between the milk in the bottle and the vacuum pump, to prevent contamination of the tubing and pump which are hard to clean. This is not achieved by the design of WO 2008/127991.

Thus, there is a need for a simple way to provide baseline vacuum in the breast pump while maintaining the separation between the milk-contaminated parts and the clean vacuum generation part of the breast pump.

US 2017/065753 A1 discloses a manual or electronic, portable fluid expression system to be used for breast milk expression from nursing women and other milk expression from any species of mammal. The fluid expression systems include a single, contiguous flexible structure designed to resemble the lips and oropharyngeal cavity of a newborn infant of the mammalian species of interest. Rigid external housings or stiffening structures can be used to mimic the bony anatomy of the infant or mammalian species of interest.

US 2021/121614 A1 discloses a breast pump for expressing human breast milk including a multipart hygiene module having a first part with a flow opening for connecting to an interior, a valve body with at least one valve and a second part with a rigid cup for forming a pump chamber. The valve body is arranged between the first and second part and the hygiene module as a whole is connectable to the housing of the breast pump in a sealing and detachable manner.

### SUMMARY OF THE INVENTION

The invention is defined by the claims.

According to examples in accordance with an aspect of the invention, there is provided a breast pump, comprising:
a breast shield for fitting over at least the nipple of a breast thereby to create a cavity over at least the nipple;
a first diaphragm between a pressure delivery inlet for connection to a pressure source and the cavity;
a valve arrangement and a second diaphragm between the cavity and the pressure delivery inlet,
wherein the valve arrangement and the second diaphragm are adapted to generate, from a pressure received at the pressure delivery inlet, a baseline pressure to be applied to the cavity.

The breast pump for example further comprises a pressure source. The pressure source, breast shield, first diaphragm and cavity outlet define the normal parts of a breast pump, for enabling a milk expression pressure profile to be applied to the breast, via the first diaphragm. The valve arrangement enables the same pressure source to be used to generate a baseline under pressure for holding the breast shield to the breast. Thus, the valve arrangement, in combination with the same pressure source and a second diaphragm, implements a secondary pump.

There is preferably an outlet from the cavity for delivering expressed milk to a milk collection container, and the valve arrangement applies a baseline pressure to the cavity via the internal volume of the milk collection container. The under pressure thus is linked to the cavity from the milk collection container (e.g. through a valve between the cavity and the milk collection container). The valve arrangement is associated with a second, diaphragm so that a separation is made between the milk contacting parts, which for example include the valve arrangement, and the pressure source (i.e. pump) and tubing. The invention thus provides an easy to implement pressure arrangement for creating a baseline under pressure, while maintaining the ability for easy cleaning. The second diaphragm may be separate to the first diaphragm, but they could instead both be formed by a single component.

An outlet valve is for example at the outlet of the cavity. Thus, the outlet valve and the valve arrangement are between the cavity and the pressure delivery inlet. The valve arrangement is between the cavity and the pressure delivery inlet, but this does not exclude other components such as this outlet valve.

The outlet valve for example leads to the milk collection container. This valve is for example a simple passive one-way valve such as a duckbill valve. It may be integrated into the first diaphragm. The outlet valve will open when the pressure in the cavity is higher than the pressure on the other side, i.e. in the milk collection container in this example. The milk collection container is then kept at a baseline pressure below the ambient pressure. In this way, the baseline pressure is transferred to the cavity to provide a holding force for holding the breast shield to the breast. It also means the flow to the milk collection container is not impeded.

A release valve is for example provided for releasing the pressure at the pressure delivery outlet of the pressure source. This is again part of the normal generation of a cyclic expression pressure waveform. It is normally used to return the pressure at the cavity to atmospheric pressure at the end of a cyclic pressure waveform. However, in this case, the pressure is only released to the baseline pressure generated by the valve arrangement.

In particular, at the pump side of the first membrane, the pressure is released to atmospheric pressure. However, because of the baseline vacuum on the other side of the first membrane, the membrane is pulled towards the cavity. The membrane has limited deformation, and this results in a retained baseline vacuum in the cavity.

The release valve for example comprises an electrically controllable valve such as a solenoid valve.

The valve arrangement for example comprises:
a first one-way valve in a forward direction from the cavity to an internal volume of the valve arrangement (e.g. via the internal volume of the milk collection container); and
a valve system for venting the internal volume of the valve arrangement to ambient surroundings,
wherein the second diaphragm is between the internal volume of the valve arrangement and the pressure delivery outlet of the pressure source.

By "forward direction" is meant that the direction in which a flow takes place through the valve from a higher pressure to a lower pressure.

The valve arrangement may simply comprise a set of passive valves. Multiple one-way valves may be combined into a single unit, for example operating in both directions with different thresholds for opening.

The second diaphragm for example has a bias to apply pressure to the internal volume of the valve arrangement. This is in the form of a pretension. It is used to create an over pressure (relative to the ambient surroundings) i.e. a positive pressure so that air can be released from the internal volume of the valve arrangement.

The valve system is for example adapted to vent the internal volume of the valve arrangement when the internal volume is at a pressure above the pressure of the ambient surroundings by a first threshold pressure or when the internal volume is at a pressure below the pressure of the ambient surroundings by at least a second threshold pressure. This second threshold pressure defines the baseline under pressure. The valve system does not allow the pressure to drop below this second threshold pressure amount lower than ambient pressure. The first threshold pressure may be zero (i.e. the valve opens as soon as there is a pressure difference) or non-zero.

The valve system for example comprises:
a second one-way valve in a forward direction from the internal volume of the valve arrangement to the ambient surroundings; and
a third one-way valve in a forward direction from the ambient surroundings to the internal volume.

The third one-way valve then comprises a pressure control valve which opens in response to a pressure difference of the second threshold pressure.

The second threshold pressure is for example in the range 1 kPa to 10 kPa, such as 2 kPa to 10 kPa.

In some embodiments, the third one-way valve is an adjustable valve that is adjustable to give a user the ability to choose the second threshold pressure.

In some embodiments, the third one-way valve is controllable to adjust the second threshold pressure based on data relevant to a physical activity of a user of the breast pump.

A controller is, for example, configured to receive the data relevant to the physical activity of the user, and control the third one-way valve to adjust the second threshold pressure based on the data.

Such a controller can be included in the breast pump itself and/or in a device, such as a smart phone or tablet computer, separate from the breast pump.

Thus, for example, operation of the third one-way valve can be linked to the user's activity levels, with the second threshold pressure being adjustable manually, electronically, and/or via an app, such as an app running on a device, such as a smart phone or tablet computer, separate from the breast pump.

The term "physical activity" refers to any voluntary bodily movement produced by skeletal muscles that requires energy expenditure.

In some embodiments, the data comprises a type of physical activity of the user, such as a type of physical activity selectable from: sitting, walking and running.

Alternatively or additionally, the data comprises an intensity level of physical activity of the user, such as an intensity level selectable from: at rest, medium activity level and high activity level.

The data can be provided in various ways, such as via a sensor, e.g. an accelerometer.

This may provide a user-friendly system because less burden is placed on the user in terms of manual interaction with the system.

Alternatively or additionally, the data includes physical activity information from a user interface configured to permit user-entry of the physical activity information. This may provide a less complex and cheaper way of providing the data relevant to the user's physical activity compared to embodiments in which the data is provided via a sensor. Also, due to the unpredictability of the user's behavior, the user-entry may be helpful in certain scenarios in which the sensor/sensory data analysis is too slow to react to the user's physical activity.

The physical activity information may, for example, be indicative of the type of physical activity being or planned to be undertaken by the user. For instance, the physical activity information comprises a selection made by the user from sitting, walking and running.

Alternatively or additionally, the physical activity information may be indicative of the intensity level of physical activity being or planned to be undertaken by the user. For instance, the physical activity information comprises a selection made by the user from rest, medium activity level and high activity level.

The user interface can, for example, be included in the breast pump itself and/or in a device, such as a smart phone or tablet computer, separate from the breast pump.

The physical activity information can be entered by the user via the user interface in various ways. For example, the user interface may include a button, e.g. a button directly placed on the breast pump. Alternatively or additionally, the user interface, e.g. button, may be provided in a remote control, such as the above-mentioned device, e.g. smart phone or tablet computer, separate from the breast pump.

In some embodiments, the data comprises a time period during which the user is to be engaged in a physical activity, with the third one-way valve being in a first state during the time period and in a second state different from the first state after termination of the time period. Thus, the user may, for instance, indicate the duration of the physical activity so that the baseline pressure returns to its original state, i.e. the second state, after a set time.

In such embodiments, the time period may be included in the physical activity information entered by the user via the user interface.

In some embodiments, the third one-way valve is adjustable to enable adjustment of the second threshold pressure during operation of the breast pump.

Thus, adjustment of the third one-way valve to adjust the second threshold pressure may permit a holding force exerted via the breast shield to be adjusted during operation of the breast pump.

Alternatively or additionally, the third one-way valve may be adjustable to adapt the second threshold pressure prior to the operation of the breast pump, e.g. based on the type and/or intensity level of physical activity planned to be undertaken by the user.

In some embodiments, preprogrammed levels of the breast pump's baseline pressure, e.g. baseline vacuum, are defined, with each preprogrammed level being associated with an intensity level of physical activity.

In such embodiments, the adjustment of the second threshold pressure based on the data relevant to the user's physical activity may include, or be defined by, selecting a preprogrammed level from said preprogrammed levels according to the intensity level of physical activity.

Alternatively or additionally, preprogrammed levels of the breast pump's baseline pressure, e.g. baseline vacuum, are defined, with each preprogrammed level being associated with a type of physical activity.

In such embodiments, the adjustment of the second threshold pressure based on the data relevant to the user's physical activity may include, or be defined by, selecting a preprogrammed level from said preprogrammed levels according to the type of physical activity.

The breast pump for example further comprises a milk collection container connected to the outlet from the cavity. The breast pump for example comprises a wearable breast pump. The baseline under pressure is thus used to hold the breast pump to the wearer to assist in hands-free operation.

The invention also provides a method of operating a breast pump for the non-therapeutic expression of milk, wherein the breast pump comprises: a pressure source; a first diaphragm between an outlet of the pressure source and a cavity over at least the nipple; a valve arrangement; and a second diaphragm between the outlet of the pressure source and the valve arrangement,
wherein the method comprises:
using the first diaphragm to transfer a pressure source pressure to the cavity for implementing a milk expression cycle; and
using the second diaphragm to transfer a baseline pressure to the internal volume of the cavity.

The baseline pressure is for example transferred to the cavity via a milk collection container.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a known breast pump system;
Figure 2 shows a wearable breast pump;
Figure 3 shows a breast pump in accordance with the invention;
Figure 4 shows the cyclic cavity pressure during use of the breast pump;
Figure 5 shows the valve states and pressure source state in a phase of the operation of the breast pump;
Figure 6 shows the valve states and pressure source state in a later phase of the operation of the breast pump;
Figure 7 shows the valve states and pressure source state in a later phase of the operation of the breast pump;
Figure 8 shows the valve states and pressure source state in a later phase of the operation of the breast pump; and
Figure 9 shows the valve states and pressure source state in a later phase of the operation of the breast pump.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a breast pump which comprises a milk expression system of a pressure source, a breast shield and a first diaphragm between a pressure delivery outlet of the pressure source and a cavity formed by the breast shield over the breast. A valve arrangement is provided, and a second diaphragm is between the outlet of the pressure source and the valve arrangement. The valve arrangement and second diaphragm are used to generate, from the pressure delivery outlet of the pressure source, a baseline pressure to be applied to the cavity, for example via the internal volume of a milk collection container. The breast shield is for receiving part of the breast and providing a seal to enable creation of an under-pressure.

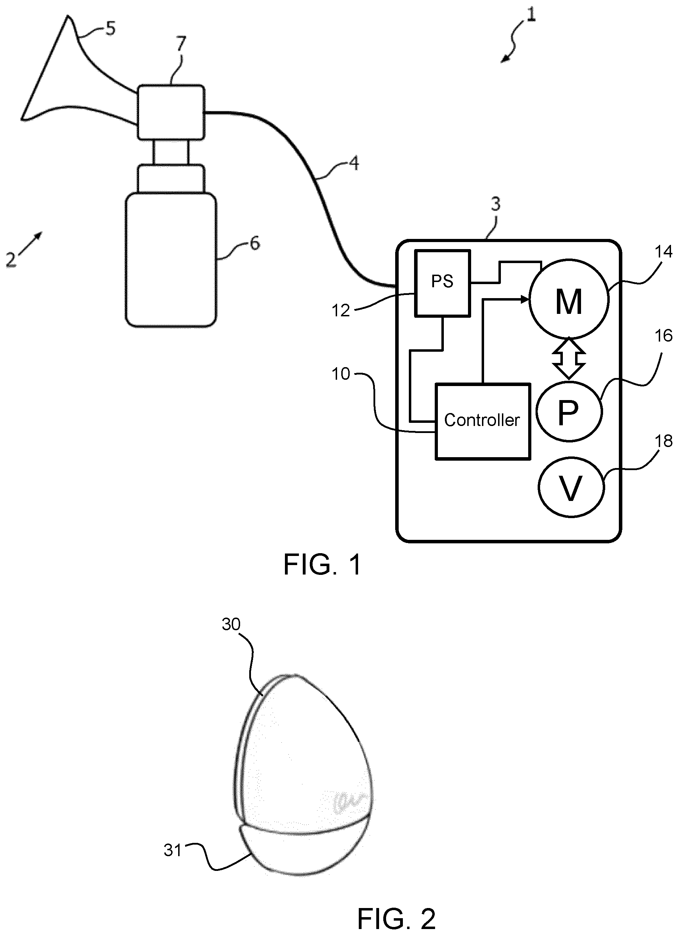

Figure 1 shows a known breast pump system 1, comprising an expression unit 2 and a drive arrangement for controlling the expression function of the breast pump.

In the example shown, the drive arrangement comprises an electric pump arrangement 3 which is connected via a tube 4 to the expression unit 2. The pump arrangement includes various components in addition to a pump impeller and the pump motor, so may be considered to be a general operating unit.

The expression unit 2 is formed with a main body 7, a funnel 5 (known as a breast shield) for receiving a breast of a user and a milk collection container 6 for collecting the expressed milk. The funnel 5 and the container 6 are connected to the main body 7. The main body 7 comprises a vacuum chamber. A flexible membrane known as the diaphragm is located in the vacuum chamber. The diaphragm prevents expressed milk from flowing into the tube 4 leading to the pump arrangement unit 3.

The operating unit, including the pump arrangement 3, may instead be directly mounted and connected to the main body 7. In this case, the diaphragm prevents expressed milk from flowing directly into the pump arrangement 3.

The pump arrangement 3 comprises a controller 10, a power source 12, a motor 14 and a vacuum pump 16 (i.e. the impeller driven by the motor 14). A membrane pump (displacement pump) may instead be used. The controller controls 10 the operation of the power source 12, motor 14 and vacuum pump 16. The pump arrangement 3 further comprises a solenoid valve 18.

In use, the vacuum pump 16 applies a vacuum to the diaphragm located in the main body 7 so that it deforms. The diaphragm deforms to create a vacuum in the funnel 5, when it is sealed to the breast, which in turn applies a vacuum to the breast which enables milk to be expressed.

The vacuum is applied to the breast at intervals. That is, a pressure differential is applied on a cyclic basis. After a vacuum has been established, the pressure from the vacuum is released by the use of the solenoid valve which is temporarily opened. The solenoid valve is an electromechanically operated valve configured to open and close an air passage that connects the vacuum side of the vacuum pump to ambient air such that when the solenoid valve is closed, the vacuum pump generates a vacuum in the expression unit which enables milk to be expressed from the breast of a user. When the solenoid valve is opened, the vacuum generated by the vacuum pump is released as ambient air flows towards the vacuum or negative pressure created by the vacuum pump such that the pressure exerted on the breast of a user is partially or completely reduced.

This is a basic description of the known operation of a standard breast pump system.

There are also wearable breast pumps, which are for example for mounting within a feeding bra. All of the drivetrain components described above are then formed within an outer enclosure which fits over the breast. A top part contains the drivetrain (pump, motor, controller) and a bottom part forms the collection container. This enables breast pumping to be performed more discretely and leaves the hands free to do other things.

There are also other types of wearable breast pump whereby the expression unit is fixed to a breast and the pump unit and/or milk collection container are situated elsewhere on the body of a user.

Figure 2 shows a wearable breast pump, comprising an expression kit 30 attached to a respective milk collection container 31.

The invention combines a standard breast pump with an additional pumping mechanism for creating a baseline vacuum, but using the already existing pressure source (i.e. pump) as the driving source, while maintaining the separation of the parts that become contaminated with milk and the vacuum generating part. In one implementation as described below, with three passive one-way valves may be used and an additional diaphragm to create the additional baseline pressure source. The baseline pressure is applied to the milk collection container (bottle) and is then transferred to the breast shield via the existing layout, in particular the existing one way valve at the inlet to the milk collection container.

The invention is of particular interest for a wearable breast pump, but it may be applied to other types of breast pump to improve ease of use or to address nipple pain or other issues.

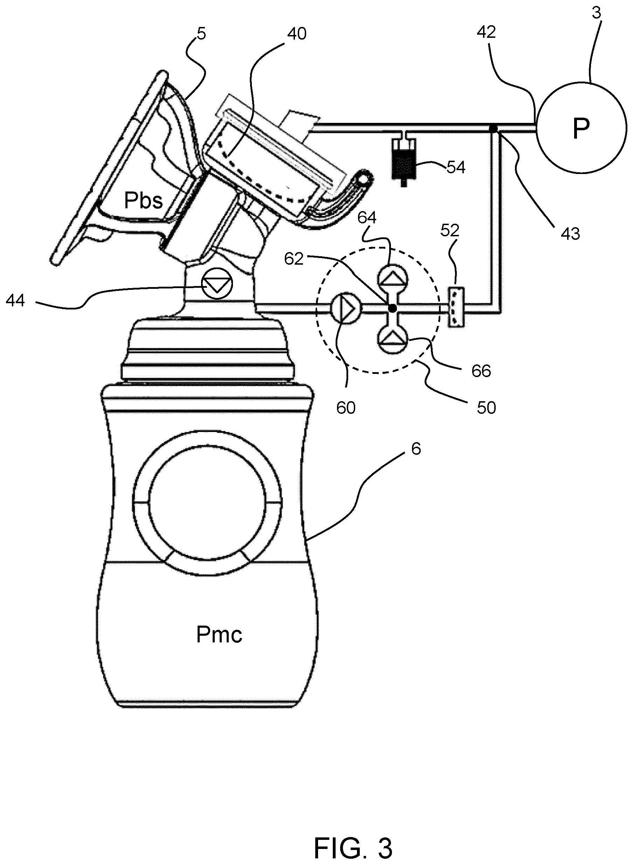

Figure 3 shows an embodiment of a breast pump, comprising a pressure source 3, and a breast shield 5 for fitting over at least the nipple of a breast thereby to create a cavity over at least the nipple. An outlet from the cavity delivers expressed milk to the milk collection container 6.

A first diaphragm 40 is positioned between a pressure delivery inlet 43 and the cavity. The pressure delivery inlet 43 is connected to a pressure delivery outlet 42 of the pressure source 3. The first diaphragm 40 means means the milk expressed into the cavity is isolated from the pressure source 3 and the conduits such as the pressure delivery outlet 42.

An outlet valve 44 is at the outlet of the cavity. This is for example a simple passive one-way valve such as a duckbill valve. It may be integrated as a part of the first diaphragm 40. The outlet valve opens when the pressure in the cavity is higher than the pressure in the milk collection container 6 and it allows milk to flow to the milk collection container.

As explained below, in another example, the outlet valve leads to a pressure buffer, and milk is collected in the cavity.

A valve arrangement 50 is provided as well as a second diaphragm 52. The two components 50,52 are between the cavity and the inlet 43 (i.e. the outlet 42 of the pressure source). The valve arrangement 50 is between the cavity and the second diaphragm 52 and the second diaphragm 52 is between the valve arrangement 50 and the inlet 43. The valve arrangement 50 and the second diaphragm 52 are together used to generate, from the pressure delivery outlet 42 of the pressure source, a baseline pressure to be applied to the internal volume of the milk collection container 6.

A release valve 54 (i.e. the valve 18 of Figure 1) is provided for releasing the pressure at the pressure delivery outlet 42 of the pressure source 3. This is again part of the normal generation of a cyclic expression pressure waveform.

The pressure source 3, breast shield 5, first diaphragm 40, release valve 54 and outlet valve 44 from the cavity define the normal parts of a breast pump, such as described above with reference to Figure 1, for enabling a milk expression pressure profile to be applied to the breast, via the first diaphragm 40.

The valve arrangement 50 and second diaphragm 52 enable the same pressure source 3 to be used to generate a baseline under pressure for holding the breast shield 5 to the breast. This under pressure is applied to the cavity via the container volume, through the valve 44. The valve arrangement is associated with its own, second, diaphragm 52 so that a separation is made between the milk contacting parts, which for example include the valve arrangement 50, and the pump 3 and pump tubing. The arrangement is thus easy to implement and easy to clean. The second diaphragm enables the pressure at the first diaphragm to be lower (i.e. a stronger vacuum) than the baseline pressure.

The release valve 54 is normally used to return the pressure at the cavity to atmospheric pressure at the end of a cyclic pressure waveform. The release valve 54 vents the pressure delivery outlet 42 to the ambient pressure. However, the cavity pressure is only released to the baseline pressure generated by the valve arrangement 50, functioning as a secondary pump. The release valve 54 for example comprises an electrically controllable valve such as a solenoid valve.

In this example, the valve arrangement comprises a first one-way valve 60 in a forward direction from the internal volume of the milk collection container to an internal volume 62 of the valve arrangement 50. That internal volume connects to the second diaphragm. Thus, the first one-way valve 60 and the second diaphragm 52 may be considered to be in series between the cavity and the pressure source 42. When the pressure in the milk collection container is above the pressure in the internal volume 62 of the valve arrangement 50, the first one-way valve 60 opens, thus pulling down the pressure in the milk collection container to the pressure in the valve arrangement. Once the system has reached a stable state, this is the baseline under pressure, as will be explained below. Whenever the normal control of the first diaphragm results in a pressure higher than the baseline pressure (i.e. closer to atmospheric pressure) the valve 44 will open so that the cavity pressure does not rise above the baseline pressure.

A second one-way valve 64 is in a forward direction from the internal volume 62 of the valve arrangement to the ambient surroundings, and a third one-way valve 66 is in a forward direction from the ambient surroundings to the internal volume. The second one-way valve opens when a first threshold pressure is present across the valve. This first threshold pressure may however be zero. The third one-way valve is a pressure control valve which only opens when there is a predetermined pressure drop of a second threshold pressure. This second threshold pressure is the baseline under pressure. If the pressure in the internal volume 62 of the valve arrangement drops below the baseline under pressure, the third one-way valve 66 opens. Thus, it prevents that the baseline under pressure becomes too low (i.e. the vacuum level is too great). The second threshold pressure is for example in the range 10mbar (1kPa) to 100mbar (10kPa), such as 20mbar (2kPa) to 100mbar (10kPa) for example 20mbar (2kPa). The third one-way valve 66 could be an adjustable valve to give the user the ability to choose the (second) threshold pressure.

The second and third one-way valves may be considered to create a valve system for venting the internal volume of the valve arrangement to ambient surroundings. This venting takes place when the pressure is above the pressure of the ambient surroundings (if the first threshold pressure is zero) or when the internal volume is at a pressure below the pressure of the ambient surroundings by more than the second threshold pressure. All three one-way valves are passive valves, meaning they need no external control but simply respond to the prevailing pressure levels. The second diaphragm 52 is between the internal volume of the valve arrangement and the pressure delivery outlet of the pressure source.

Different valve arrangements may of course be used to implement the functionality as explained above. For example, the valve system (of second and third valves) may be a single valve with the desired threshold behavior. Thus, they may combined in one part with two valve functions.

The second diaphragm for example has a pretension to create a bias to apply pressure to the internal volume 62 of the valve arrangement. It is used to enable air to be released from the internal volume and overcome the opening pressure of the second one-way valve 64.

The pressure in the milk collection container may be defined as Pmc and the pressure in the breast shield may be defined as Pbs (i.e. the cavity pressure).

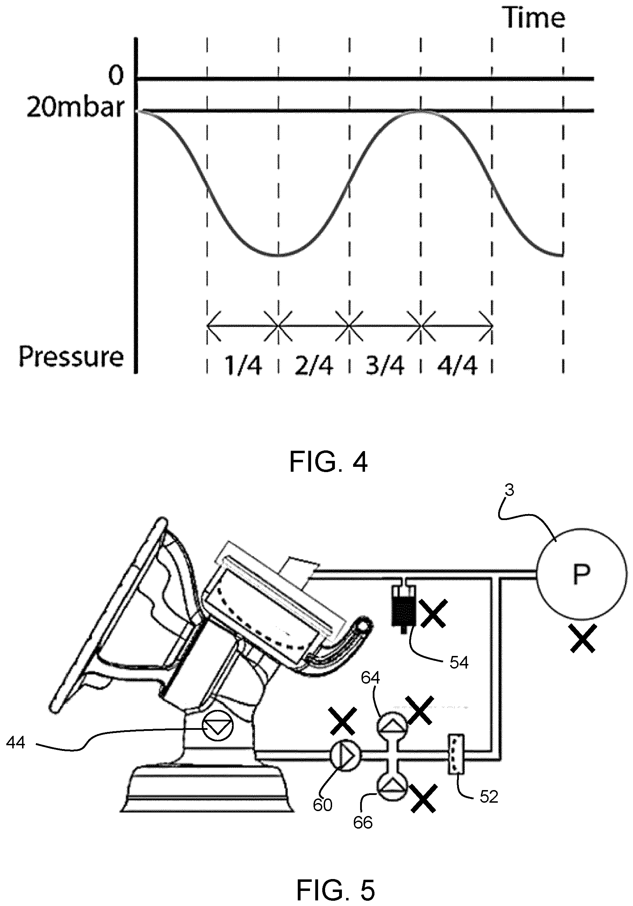

Figure 4 shows the cavity pressure Pbs over time. It shows the cyclic expression waveform, but with a 20mbar (2kPa) baseline under pressure. The vacuum profile within the breast shield thus oscillates from a maximum negative pressure to the pressure in the container Pmc and does not reach the atmospheric pressure. No special modification is necessary to a standard pump design. Instead, when the pump is returning back to atmospheric pressure in conventional manner, a baseline pressure is retained in the cavity of the breast shield. The first diaphragm 40 between the pump and the breast shield 5 means the pump can return to atmospheric pressure while the breast shield chamber pressure Pbs can remain lower than atmospheric pressure by means of the valve arrangement 50. The main valve 44 (e.g. duckbill valve) at the outlet of the cavity will automatically close when Pbs<Pmc. Thus, the oscillating negative pressure only reaches the breast shield cavity and the part of the vacuum channels at the pump-side of the first and second diaphragms.

Figure 5 shows the status of the valves and pump when first applying the breast pump. The milk collection container pressure Pmc is equal to the pressure in the breast shield Pbs (i.e. the cavity pressure) and equal to atmospheric pressure (Pbs=Pmc=Patm). The pump is off and all valves are closed since there are no pressure differences.

Once the breast pump is applied with the breast in the funnel and the pressure source 3 is turned on, an oscillating negative pressure is created, and the breast shield pressure Pbs oscillates. The aim is for the valve arrangement 50 to create an under pressure in the container Pmc that remains constant.

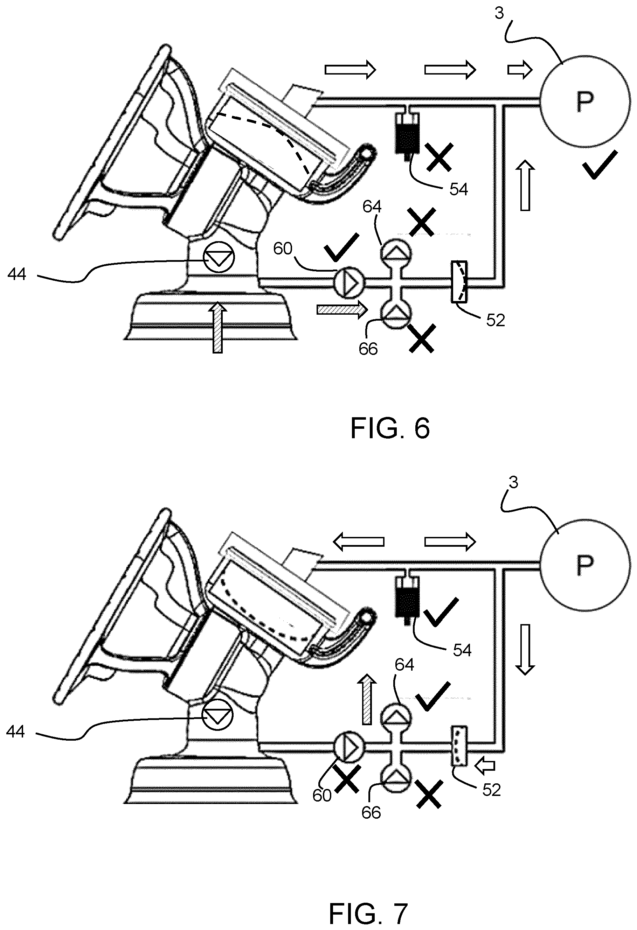

Figure 6 shows the status of the valves and pump when first applying the cyclic pressure waveform. An under pressure is built up in the part of the system that is behind the diaphragms. At the first diaphragm, this under pressure is directly transferred to the cavity within the breast shield. The movement of the second diaphragm increases the internal volume of the valve arrangement and this generates an under pressure which opens the first one-way valve 60 between the second diaphragm 52 and the milk collection container volume.

Figure 7 shows the status of the valves and pump when first releasing the cyclic pressure waveform. The release valve 54 is opened, by electronic control. The pressure at the pump side of the two diaphragms is released to the atmospheric pressure. Because of the pretension of the second diaphragm as mentioned above, air is pushed out of the internal volume of the valve arrangement through the second one-way valve 64 (e.g. a duckbill valve).

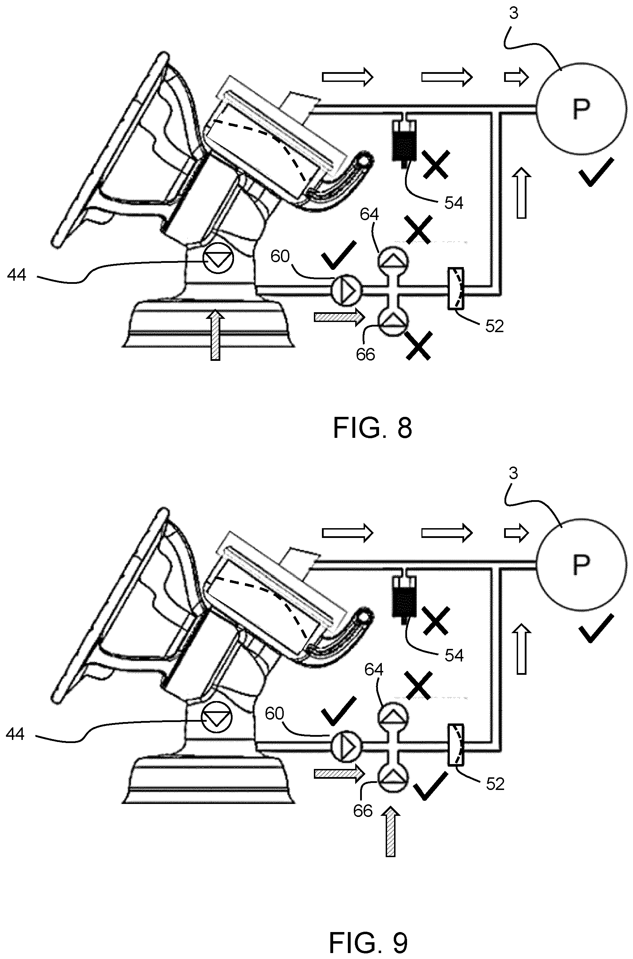

The application of pressure is then repeated, as shown in Figure 8. This is identical to Figure 6.

After a few cycles, the baseline under pressure level will be reached in the internal volume of the valve arrangement. The third one-way valve 66 then opens. It functions as a pressure control valve and it creates a controlled leakage to prevent the valve arrangement delivering a deeper vacuum (i.e. lower absolute pressure) than the desired baseline vacuum level.

At the end of a breast pumping session, it will be desirable to release the baseline pressure so that the breast shield may more easily be removed.

There are various options for this, such as a user-operated release valve (e.g. at the top of the coupling to the milk collection container). One option is for the user to a put a finger between the funnel and breast (as a mother would do with a baby that needs to be removed from the breast)

, Alternatively, the user may be able to manually actuate (e.g. squeeze) valves, for example valves 44 and 60 and one of 64 and 66 may be opened. This is for example possible if they are formed as a parts of single unit, and a push button may then be provided to perform the release function.

The breast pump for example comprises a wearable breast pump.

In the example above, the baseline pressure is delivered to the cavity via the milk collection container. However, in another example, the two-diaphragm design and valve arrangement may be used to apply the baseline pressure to the cavity via a pressure vessel that is separate to the milk collection container (again with valve 44 between the cavity and the pressure vessel). The milk collection container may for example simply comprise the cavity itself.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A breast pump, comprising:
a breast shield (5) for fitting over at least the nipple of a breast thereby to create a cavity over at least the nipple;
a first diaphragm (40) between a pressure delivery inlet (43), for connection to a pressure source (3), and the cavity;
a valve arrangement (50) and a second diaphragm (52) between the cavity and the pressure delivery inlet (43),
wherein the valve arrangement (50) and the second diaphragm (52) are adapted to generate, from a pressure received at the pressure delivery inlet (43), a baseline pressure to be applied to the cavity, the valve arrangement (50) comprising a first one-way valve (60) in a forward direction from the cavity to an internal volume (62) of the valve arrangement; and a valve system (64,66) for venting the internal volume (62) of the valve arrangement to ambient surroundings,
wherein the valve system (64,66) is adapted to vent the internal volume (62) of the valve arrangement when the internal volume is at a pressure above the pressure of the ambient surroundings by at least a first threshold pressure or when the internal volume is at a pressure below the pressure of the ambient surroundings by at least a second threshold pressure, and
wherein the second diaphragm (52) is between the internal volume (62) of the valve arrangement (50) and the pressure delivery inlet (43).

2. The breast pump of claim 1, further comprising a pressure source having a pressure outlet for connection to the pressure delivery inlet, and a milk collection container (6) connected to an outlet from the cavity.

3. The breast pump of claim 2, wherein the cavity has an outlet for delivering milk to the milk collection container, and the valve arrangement and the second diaphragm apply the baseline pressure to the cavity via an internal volume of the milk collection container.

4. The breast pump of claim 3, wherein the cavity outlet has an outlet valve (44).

5. The breast pump of any one of claims 1 to 4, comprising a release valve (54) for releasing the pressure at the pressure delivery inlet (43).

6. The breast pump of claim 5, wherein the release valve (54) comprises an electrically controllable valve.

7. The breast pump of any one of claims 1 to 6, wherein the second diaphragm (52) has a bias to apply positive pressure to the internal volume (62) of the valve arrangement.

8. The breast pump of any one of claims 1 to 7, wherein the valve system (64,66) comprises:
a second one-way valve (64) in a forward direction from the internal volume (62) of the valve arrangement to the ambient surroundings; and
a third one-way valve (66) in a forward direction from the ambient surroundings to the internal volume (62).

9. The breast pump of claim 8, wherein the first to third one-way valves (60, 64, 66) are passive valves.

10. The breast pump of claim 8 or 9, wherein the third one-way valve (66) is an adjustable valve that is adjustable to give a user the ability to choose the second threshold pressure.

11. The breast pump of any one of claims 8 to 10, wherein the third one-way valve (66) is controllable to adjust the second threshold pressure based on data relevant to a physical activity of a user of the breast pump; optionally wherein the data comprises a type of physical activity of the user and/or an intensity level of physical activity of the user.

12. The breast pump of any one of claims 8 to 11, wherein the third one-way valve (66) comprises a pressure control valve which opens in response to a pressure difference equal to the second threshold pressure.

13. The breast pump of any one of claims 1 to 12, wherein the second threshold pressure is in the range 1 kPa to 10 kPa.

14. The breast pump of any one of claims 1 to 13, comprising a wearable breast pump.

15. A method of operating a breast pump according to any of claims 1-14,
wherein the method comprises:
using the first diaphragm to transfer a pressure source pressure from the pressure source to set the breast pump into a state which, when the cavity is created, permits implementing of a milk expression cycle,
wherein setting the breast pump into said state further comprises using the second diaphragm to transfer a baseline pressure from the pressure source, wherein the method does not include operation of the breast pump to cause expression of milk.

## Patentansprüche

1. Milchpumpe, umfassend:
ein Brustschild (5) zum Einpassen über mindestens die Brustwarze einer Brust, um dadurch einen Hohlraum über mindestens der Brustwarze zu kreieren;
ein erstes Diaphragma (40) zwischen einem Druckzufuhreinlass (43) zur Verbindung mit einer Druckquelle (3) und dem Hohlraum;
eine Ventilanordnung (50) und ein zweites Diaphragma (52) zwischen dem Hohlraum und dem Druckzufuhreinlass (43),
wobei die Ventilanordnung (50) und das zweite Diaphragma (52) adaptiert sind, um aus einem an dem Druckzufuhreinlass (43) empfangenen Druck einen auf den Hohlraum anzuwendenden Basisdruck zu erzeugen, wobei die Ventilanordnung (50) ein erstes Einwegventil (60) in einer Vorwärtsrichtung von dem Hohlraum zu einem internen Volumen (62) der Ventilanordnung umfasst; und ein Ventilsystem (64, 66) zum Entlüften des internen Volumens (62) der Ventilanordnung in die umgebende Umgebung,
wobei das Ventilsystem (64, 66) adaptiert ist, um das interne Volumen (62) der Ventilanordnung zu entlüften, wenn das interne Volumen bei einem Druck ist, der mindestens um einen ersten Schwellendruck über dem Druck der umgebenden Umgebung ist, oder wenn das interne Volumen bei einem Druck ist, der mindestens um einen zweiten Schwellendruck unter dem Druck der umgebenden Umgebung ist, und
wobei das zweite Diaphragma (52) zwischen dem internen Volumen (62) der Ventilanordnung (50) und dem Druckzufuhreinlass (43) ist.

2. Milchpumpe nach Anspruch 1, weiter umfassend eine Druckquelle, die einen Druckauslass zur Verbindung mit dem Druckzufuhreinlass aufweist, und einen Milchsammelbehälter (6), der mit einem Auslass aus dem Hohlraum verbunden ist.

3. Milchpumpe nach Anspruch 2, wobei der Hohlraum einen Auslass zum Zuführen von Milch an den Milchsammelbehälter aufweist und die Ventilanordnung und das zweite Diaphragma den Basisdruck über ein internes Volumen des Milchsammelbehälters auf den Hohlraum anwenden.

4. Milchpumpe nach Anspruch 3, wobei der Hohlraumauslass ein Auslassventil (44) aufweist.

5. Milchpumpe nach einem der Ansprüche 1 bis 4, umfassend ein Ablassventil (54) zum Ablassen des Drucks am Druckzufuhreinlass (43).

6. Milchpumpe nach Anspruch 5, wobei das Ablassventil (54) ein elektrisch steuerbares Ventil umfasst.

7. Milchpumpe nach einem der Ansprüche 1 bis 6, wobei das zweite Diaphragma (52) eine Vorspannung aufweist, um einen Überdruck auf das interne Volumen (62) der Ventilanordnung anzuwenden.

8. Milchpumpe nach einem der Ansprüche 1 bis 7, wobei das Ventilsystem (64, 66) Folgendes umfasst:
ein zweites Einwegventil (64) in einer Vorwärtsrichtung vom internen Volumen (62) der Ventilanordnung zur umgebenden Umgebung; und
ein drittes Einwegventil (66) in einer Vorwärtsrichtung von der umgebenden Umgebung zu dem internen Volumen (62).

9. Milchpumpe nach Anspruch 8, wobei das erste bis dritte Einwegventil (60, 64, 66) passive Ventile sind.

10. Milchpumpe nach Anspruch 8 oder 9, wobei das dritte Einwegventil (66) ein anpassbares Ventil ist, das anpassbar ist, um einem Benutzer die Fähigkeit zu geben, den zweiten Schwellendruck zu wählen.

11. Milchpumpe nach einem der Ansprüche 8 bis 10, wobei das dritte Einwegventil (66) steuerbar ist, um den zweiten Schwellendruck basierend auf Daten anzupassen, die für eine physische Aktivität eines Benutzers der Milchpumpe relevant sind; wobei die Daten optional eine Art der physischen Aktivität des Benutzers und/oder ein Intensitätslevel der physischen Aktivität des Benutzers umfassen.

12. Milchpumpe nach einem der Ansprüche 8 bis 11, wobei das dritte Einwegventil (66) ein Drucksteuerventil umfasst, das sich als Antwort auf eine Druckdifferenz öffnet, die gleich dem zweiten Schwellendruck ist.

13. Milchpumpe nach einem der Ansprüche 1 bis 12, wobei der zweite Schwellendruck im Bereich von 1 kPa bis 10 kPa ist.

14. Milchpumpe nach einem der Ansprüche 1 bis 13, umfassend eine tragbare Milchpumpe.

15. Verfahren zum Betreiben einer Milchpumpe nach einem der Ansprüche 1 bis 14, wobei das Verfahren Folgendes umfasst:
Verwenden des ersten Diaphragmas, um einen Druckquellendruck von der Druckquelle zu übertragen, um die Milchpumpe in einen Zustand zu versetzen, der, wenn der Hohlraum kreiert ist, Implementieren eines Milchexprimierungszyklus gestattet,
wobei das Versetzen der Milchpumpe in den Zustand weiter Verwenden des zweiten Diaphragmas zum Übertragen eines Basisdrucks von der Druckquelle umfasst, wobei das Verfahren nicht den Betrieb der Milchpumpe zum Verursachen der Exprimierung von Milch einschließt.

## Revendications

1. Tire-lait, comprenant :
une téterelle (5) destinée à être placée sur au moins le mamelon d'un sein, ce qui permet de créer une cavité au-dessus au moins du mamelon ;
un premier diaphragme (40) entre une entrée de distribution de pression (43) pour une connexion à une source de pression (3), et la cavité ;
un agencement de soupape (50) et un second diaphragme (52) entre la cavité et l'entrée de distribution de pression (43),
dans lequel l'agencement de soupape (50) et le second diaphragme (52) sont adaptés pour générer, à partir d'une pression reçue au niveau de l'entrée de distribution de pression (43), une pression de base à appliquer à la cavité, l'agencement de soupape (50) comprenant une première soupape unidirectionnelle (60) dans une direction vers l'avant depuis la cavité vers un volume interne (62) de l'agencement de soupape ; et un système de soupape (64, 66) pour ventiler le volume interne (62) de l'agencement de soupape dans l'environnement ambiant,
dans lequel le système de soupape (64, 66) est adapté pour ventiler le volume interne (62) de l'agencement de soupape lorsque le volume interne est à une pression supérieure à la pression de l'environnement ambiant d'au moins une première pression de seuil ou lorsque le volume interne est à une pression inférieure à la pression de l'environnement ambiant d'au moins une seconde pression de seuil, et
dans lequel le second diaphragme (52) se trouve entre le volume interne (62) de l'agencement de soupape (50) et l'entrée de distribution de pression (43).

2. Tire-lait selon la revendication 1, comprenant en outre une source de pression présentant une sortie de pression pour un raccordement à l'entrée de distribution de pression et un récipient de collecte de lait (6) raccordé à une sortie de la cavité.

3. Tire-lait selon la revendication 2, dans lequel la cavité présente une sortie pour distribuer du lait au récipient de collecte de lait, et l'agencement de soupape et le second diaphragme appliquent la pression de base à la cavité via un volume interne du récipient de collecte de lait.

4. Tire-lait selon la revendication 3, dans lequel la sortie de cavité présente une soupape de sortie (44).

5. Tire-lait selon l'une quelconque des revendications 1 à 4, comprenant une soupape de décharge (54) pour libérer la pression au niveau de l'entrée de distribution de pression (43).

6. Tire-lait selon la revendication 5, dans lequel la soupape de décharge (54) comprend une soupape à commande électrique.

7. Tire-lait selon l'une quelconque des revendications 1 à 6, dans lequel le second diaphragme (52) présente une polarisation pour appliquer une pression positive au volume interne (62) de l'agencement de soupape.

8. Tire-lait selon l'une quelconque des revendications 1 à 7, dans lequel le système de soupape (64, 66) comprend :
une deuxième soupape unidirectionnelle (64) dans une direction vers l'avant depuis le volume interne (62) de l'agencement de soupape vers l'environnement ambiant ; et
une troisième soupape unidirectionnelle (66) dans une direction vers l'avant depuis l'environnement ambiant vers le volume interne (62).

9. Tire-lait selon la revendication 8, dans lequel les première à troisième soupapes unidirectionnelles (60, 64, 66) sont des soupapes passives.

10. Tire-lait selon la revendication 8 ou 9, dans lequel la troisième soupape unidirectionnelle (66) est une soupape ajustable qui peut être ajustée pour donner à une utilisatrice la possibilité de choisir la seconde pression de seuil.

11. Tire-lait selon l'une quelconque des revendications 8 à 10, dans lequel la troisième soupape unidirectionnelle (66) peut être commandée pour ajuster la seconde pression de seuil sur la base de données relatives à une activité physique d'une utilisatrice du tire-lait ; facultativement, dans lequel les données comprennent un type d'activité physique de l'utilisatrice et/ou un niveau d'intensité d'activité physique de l'utilisatrice.

12. Tire-lait selon l'une quelconque des revendications 8 à 11, dans lequel la troisième soupape unidirectionnelle (66) comprend une soupape de commande de pression qui s'ouvre en réponse à une différence de pression égale à la seconde pression de seuil.

13. Tire-lait selon l'une quelconque des revendications 1 à 12, dans lequel la seconde pression de seuil est dans la plage allant de 1 kPa à 10 kPa.

14. Tire-lait selon l'une quelconque des revendications 1 à 13, comprenant un tire-lait portable.

15. Procédé de fonctionnement d'un tire-lait selon l'une quelconque des revendications 1-14, dans lequel le procédé comprend :
l'utilisation du premier diaphragme pour transférer une pression de source de pression à partir de la source de pression pour régler le tire-lait dans un état qui, lorsque la cavité est créée, permet la mise en œuvre d'un cycle d'expression de lait,
dans lequel le réglage du tire-lait dans ledit état comprend en outre l'utilisation du second diaphragme pour transférer une pression de base à partir de la source de pression, dans lequel le procédé n'inclut pas le fonctionnement du tire-lait pour provoquer une expression de lait.
